# EUROPEAN PATENT APPLICATION

(11) **EP 4 512 848 A1**
(43) Date of publication of application: **26.02.2025**
(21) Application number: 23791125.0
(22) Date of filing: 13.04.2023
(51) Int. Cl.: C08G 77/20, C08G 77/12, C08G 77/04, C08L 83/04, C08L 83/05, C08L 83/07, C09J 183/04, C09J 183/05, C09J 183/07, A61K 8/00

(54) **CURABLE SILOXANE COMPOSITION AND USE THEREOF IN SKIN CARE PRODUCTS**

(30) Priority: 20.04.2022 CN 202210419302
(71) Applicant: DuPont Electronics, Inc., Wilmington, DE 19805 (US); DuPont Technology (Shanghai) Co., Ltd., Shanghai, 200131 (CN)
(72) Inventor: YANG, Zheyue, Shanghai 201203 (CN); SHI, Yuan, Shanghai 201203 (CN); QI, Xiaoman, Shanghai 201203 (CN); WU, Qiuju, Shanghai 201203 (CN); LYU, Jian, Shanghai 201203 (CN)
(74) Representative: Abitz & Partner
(86) International application number: PCT/CN2023/088231
(87) International publication number: WO 2023/202470

(57) **Abstract**

A curable silicone composition and the use thereof in skin care products. Provided is a curable silicone composition comprising: (a) based on the total weight of the curable siloxane composition, an organosiloxane component having an alkenyl content of 0.01-2 mol/kg; (b) based on the total weight of the curable siloxane composition, an organosiloxane component with the content of hydrogen of silicon-hydrogen bonds being 0.01-2 mol/kg; and (c) a curing catalyst, wherein the molar ratio of alkenyl to hydrogen of silicon-hydrogen bonds is 0.8 or greater.

## Description

### Technical Field

The present application relates to a curable siloxane composition, and in particular to a curable siloxane composition for skin (e.g., human skin) care articles.

### Background Art

Although skin care products represent a huge and rapidly growing market, there is currently a lack of products for skin care taking advantage of sleeping time. At present, the skin care products on the market are water-based, which means they dry out quickly with a short caring duration. Generally, the existing skin care products only work for 20 minutes. In addition, existing skin care products require the customer to remain motionless during application because skin care products generally contain liquid (e.g., water), which easily escapes when people move.

Accordingly, there is a need for a care product that can be comfortably attached to the skin for an extended period and removed from the skin without causing pain or discomfort while providing skincare functions (e.g., moisturization).

### Summary of the Invention

An aspect of the present application provides a curable siloxane composition, comprising: (a) based on the total weight of the curable siloxane composition, an organosiloxane component having an alkenyl content of 0.01-2 mol/kg; (b) based on the total weight of the curable siloxane composition, an organosiloxane component with the content of hydrogen of silicon-hydrogen bonds being 0.01-2 mol/kg; and (c) a curing catalyst, wherein the molar ratio of alkenyl to Si-H is 0.8 or greater.

### Brief Description of the Drawings

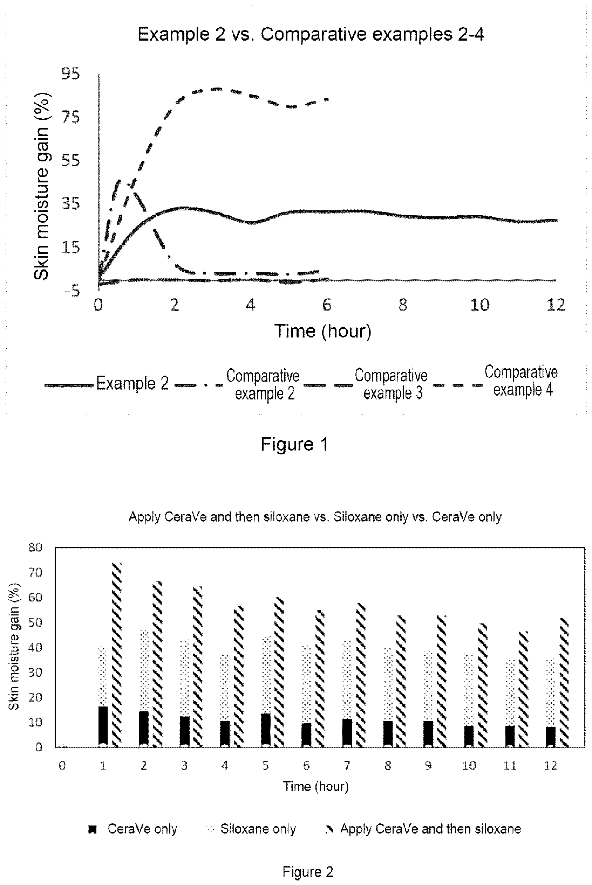

FIG. 1 illustrates a comparison of the effect of Example 2 with Comparative examples 2-4.
**FIG.** 2 illustrates the synergistic effect of Example 2 and CeraVe.

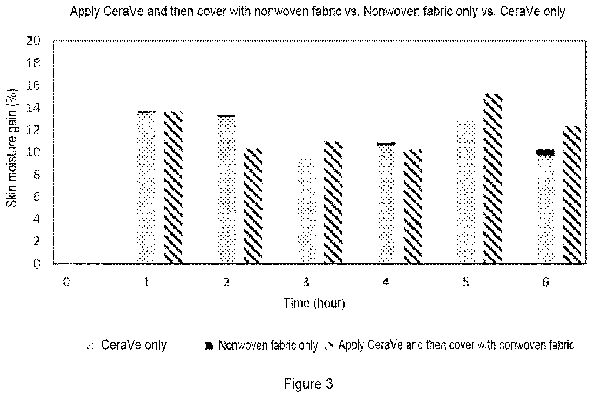

FIG. 3 illustrates the effects of Comparative example 3 and CeraVe.

### Detailed Description of Embodiments

In the present disclosure, unless otherwise specified, percentages (%) or parts refer to weight percentages or parts by weight relative to the composition.

In the present disclosure, unless otherwise specified, the components involved or the preferred components thereof can be combined with one another to form new technical solutions.

In the present disclosure, unless otherwise specified, all the embodiments and preferred embodiments mentioned herein can be combined with one another to form new technical solutions.

In the present disclosure, unless otherwise specified, all the technical features and preferred features mentioned herein can be combined with one another to form new technical solutions.

In the present disclosure, in the absence of any statement to the contrary, the sum of the contents of the components in the composition is 100%.

In the present disclosure, in the absence of any statement to the contrary, the sum of the parts of the components in the composition may be 100 parts by weight.

In the present disclosure, unless stated otherwise, the numerical range "a-b" denotes an abbreviated representation of any combination of real numbers between a and b, where both a and b are real numbers. For example, the numerical range "0-5" means that all the real numbers between "0-5" have been listed herein, and "0-5" is just an abbreviated representation of combinations of these numerical values.

In the present disclosure, unless stated otherwise, the integer value range "a-b" denotes an abbreviated representation of any combination of integers between a and b, where both a and b are integers. For example, the integer value range "1-N" denotes 1, 2, ..., and N, where N is an integer.

In the present disclosure, unless stated otherwise, "combination thereof" denotes a multi-component mixture of said elements, for example, a multi-component mixture of two, three, four, and up to the maximum possible.

Unless otherwise specified, the term "a/an" used in the present description refers to "at least one".

Unless otherwise specified, the percentages (including weight percentages) described in the present disclosure are all based on the total weight of the composition.

The "range" disclosed herein is in the form of a lower limit and an upper limit. There may be one or a plurality of lower limits and one or a plurality of upper limits, respectively. A given range is defined by selecting a lower limit and an upper limit. The selected lower and upper limits define the boundaries of a specific range. All ranges that can be defined in this way are inclusive and combinable, that is to say, any lower limit can be combined with any upper limit to form a range. For example, if the ranges 60-120 and 80-110 are listed for a particular parameter, it should be understood that the ranges 60-110 and 80-120 are also to be expected. Additionally, if the minimum values listed for a range are 1 and 2, and the maximum values listed for the range are 3, 4, and 5, the following ranges all can be expected: 1-3, 1-4, 1-5, 2-3, 2-4, and 2-5.

Herein, unless otherwise stated, all reactions are carried out at normal temperature and pressure.

Herein, unless otherwise stated, the reaction steps may be performed sequentially or non in order. For example, additional steps could be included between the reaction steps, and the sequence of the reaction steps could be changed. Preferably, the reaction method herein is carried out sequentially.

Herein, unless otherwise stated, the terms "comprise", "include", etc., denote that any other suitable component/monomer may also be included in addition to the listed component/monomer.

An aspect of the present application provides a curable siloxane composition, comprising: (a) based on the total weight of the curable siloxane composition, an organosiloxane component having an alkenyl content of 0.01-2 mol/kg; (b) based on the total weight of the curable siloxane composition, an organosiloxane component with the content of hydrogen of silicon-hydrogen bonds being 0.01-2 mol/kg; and (c) a curing catalyst, wherein the molar ratio of alkenyl to hydrogen of silicon-hydrogen bonds is 0.8 or greater.

The organosiloxane component (a) may be of any structure. The organosiloxane component (a) may be a linear or branched oligomer or polymer. The organosiloxane component (a) contains at least 0.01-2 mol/kg (generally 0.05-1.5 mol/kg, preferably 0.05-1.0 mol/kg, more preferably 0.08-0.5 mol/kg) of alkenyl per molecule, and the alkenyl is typically covalently linked to a silicon atom in the organosiloxane. Examples of alkenyl groups include vinyl, allyl, butenyl, pentenyl, and hexenyl groups. They may be side chains or terminals or combinations thereof, that is to say, they may be present on any of the silyloxy units of the organosiloxane component (a).

In an example of the present application, the organosiloxane component (a) may be of the formula below:

(R₁R₂R₃)Si-O-[Si(R₄)(R₅)O]ₘ-[Si(CH₃)(CH=CH₂)O]ₙ-Si(CH₃)(CH₃)(C=CH₂) (I)

In the formula, R₁-R₅ are each independently selected from alkyl having 1-10 carbon atoms (e.g.,1-6 carbon atoms, 1-4 carbon atoms), hydroxyl, aryl (e.g., phenyl or biphenyl) having 6-20 carbon atoms (e.g., 6-12 carbon atoms, 6-10 carbon atoms), alkenyl having 2-10 carbon atoms (e.g., 2-6 carbon atoms, 2-4 carbon atoms), alkoxy having 1-10 carbon atoms (e.g., 1-6 carbon atoms, 1-4 carbon atoms), HON=C-, CH₂(O)CHCH₂OCH₂CH₂CH₂-, -CH₂CH₂CH₂NH₂, - CH₂CH₂CH₂NHCH₂CH₂NH₂, and -OC(O)CH₃; and m and n each independently represent 0-2000, for example, 0-1000, preferably 1-500, and more preferably 1-200.

Examples of the organosiloxane component (a) that may be used include vinyldimethylsilyloxy-terminated dimethylsiloxane - vinylmethylsiloxane copolymer, vinyldimethylsilyloxy-terminated polydimethylsiloxane, vinylphenylmethylsiloxy-terminated dimethylsiloxane - vinylmethylsiloxane copolymer, and mixtures thereof.

The organosiloxane component (b) may be a single polymer or oligomer, or a combination of two or more different polymers or oligomers.

The organosiloxane component (b) may be of any structure. The organosiloxane component (b) may be a linear or branched oligomer or polymer. The organosiloxane component (b) contains at least 0.01-2 mol/kg (generally 0.05-1.5 mol/kg, preferably 0.05-1.0 mol/kg, more preferably 0.08-0.5 mol/kg) of hydrogen of silicon-hydrogen bonds per molecule. The hydrogens of silicon-hydrogen bonds may be side chains or terminals or combinations thereof, that is to say, they may be present on any of the silyloxy units of the organosiloxane component (b).

In an example of the present application, the organosiloxane component (b) may be of the formula below:

(R₆R₇R₈)Si-O-[Si(R₉)(R₁₀)O]ₐ-[Si(CH₃)(H)O]_{b}-Si(CH₃)(CH₃)(H) (II)

In the formula, R₆-R₁₀ are each independently selected from alkyl having 1-10 carbon atoms (e.g.,1-6 carbon atoms, 1-4 carbon atoms), H, hydroxyl, aryl (e.g., phenyl or biphenyl) having 6-20 carbon atoms (e.g., 6-12 carbon atoms, 6-10 carbon atoms), alkoxy having 1-10 carbon atoms (e.g., 1-6 carbon atoms, 1-4 carbon atoms), HON=C-, CH₂(O)CHCH₂OCH₂CH₂CH₂-, - CH₂CH₂CH₂NH₂, -CH₂CH₂CH₂NHCH₂CH₂NH₂, and -OC(O)CH₃; and a and b each independently represent 0-2000, for example, 0-1000, preferably 1-500, and more preferably 1-200.

Examples of the organosiloxane component (b) that may be used include hydrodimethylsiloxy-terminated dimethylsiloxane - hydromethylsiloxane copolymer, hydrodimethylsiloxy-terminated polydimethylsiloxane, hydromethylethylsiloxy-terminated dimethylsiloxane - hydromethylsiloxane copolymer, and mixtures thereof.

The organosiloxane component (b) may be a single polymer or oligomer, or a combination of two or more different polymers or oligomers.

Typically, the molar ratio of alkenyl to hydrogen of silicon-hydrogen bonds in the curable siloxane composition is 0.8 or greater, for example, 0.8 : 1 - 10 : 1, preferably 0.8 : 1 - 8 : 1, more preferably 1 : 1 - 5 : 1, and most preferably 1 : 1 - 2 : 1, for example 1 : 1 - 1.5 : 1 or 0.8 : 1 - 1.2 : 1.

The viscosity at 25°C of the organosiloxane composition before curing is typically in a range of 0.1 Pa.s to 100 Pa.s. Unless otherwise stated, all viscosities are measured with a rotation viscometer such as a Brookfield viscometer, or using a rheometer.

The curing catalyst (c) in the curable siloxane composition of the present application may be any catalyst used for curing organosiloxanes. The catalyst is well known in the art and includes, for example, a catalyst selected from platinum group metals or transition metals of the periodic table of the elements, such as platinum, ruthenium, rhodium, palladium, osmium, and iridium; and compounds thereof. In the present application, the catalyst may be selected from platinum-based catalysts, such as chloroplatinic acid, chloroplatinic acid dissolved in alcohols or ketones and matured solutions thereof, platinum-olefin complexes, chloroplatinic acid-alkenylsiloxane complexes, chloroplatinic acid-diketone complexes, platinum black, carrier-loaded platinum, and mixtures thereof.

The amount of the curing catalyst (c) should be sufficient to cure the organosiloxane component (a) and the organosiloxane component (b) in the composition. For example, it may be added to the curable composition in such an amount that,based on the total weight of the organosiloxane components (a) and (b), 0.1 ppm by weight to 500 ppm by weight, or 1 ppm by weight to 200 ppm by weight, or 1 ppm by weight to 100 ppm by weight of platinum atoms are provided.

The curable composition of the present application may further comprise an inhibitor, for example, an inhibitor for platinum-based catalysts. The inhibitor includes, but is not limited to, hydrazines, triazoles, phosphines, thiols, organonitrogen compounds, acetylenic alcohols, silylated acetylenic alcohols, maleates, fumarates, ethylenically or aromatic unsaturated amides, ethylenically unsaturated isocyanate, olefinic siloxanes, unsaturated hydrocarbon monoesters and diesters, conjugated ene-ynes, hydroperoxides, nitriles, and diaziridines.

The inhibitor may be selected from acetylenic alcohols containing at least one unsaturated bond and derivatives thereof. The examples of the acetylenic alcohols and derivatives thereof include 1-ethynyl-1-cyclohexanol (ETCH), 2-methyl-3-butyn-2-ol, 3-butyn-1-ol, 3-butyn-2-ol, propargyl alcohol, 2-phenyl-2-propyn-1-ol, 3,5-dimethyl-1-hexyn-3-ol, 1-ethynylcyclopentanol, 1-phenyl-2-propynol, 3-methyl-1-penten-4-yn-3-ol, and mixtures thereof.

Preferably, the inhibitor is selected from 1-ethynyl-1-cyclohexanol, 2-methyl-3-butyn-2-ol, 3-butyn-1-ol, 3-butyn-2-ol, propargyl alcohol, 2-phenyl -2-propyn-1-ol, 3,5-dimethyl-1-hexyn-3-ol, 1-ethynylcyclopentanol, 1-phenyl-2-propynol, and mixtures thereof.

Typically, the amount of the inhibitor in the curable siloxane composition is 10 ppm by weight to 50,000 ppm by weight. Alternatively, the inhibitor is present in such an amount that the molar ratio of the inhibitor to the catalytically active atoms (e.g., platinum atoms) in the catalyst is 150 to 900, or 150 to 700, or 150 to 600.

The curable siloxane composition of the present application may further comprise an additive commonly used in the art. Examples of the additive include fillers, flame retardants, pigments, lubricants, adhesion promoters, mold release agents, diluents, solvents, UV light stabilizers, biocides, wetting agents, heat stabilizers, plasticizers and the like.

Examples of fillers include quartz powder, diatomaceous earth, talc, clay, calcium carbonate, magnesium carbonate, hollow glass, glass fiber, hollow resin and plated powder, and mixtures or derivatives thereof.

Examples of chain extenders include linear organopolysiloxanes containing 2 hydrogen groups bonded to silicon on the terminal position.

Examples of flame retardants include aluminum trihydrate, chlorinated paraffins, hexabromocyclododecane, triphenyl phosphate, dimethyl methylphosphonate, tris(2,3-dibromopropyl) phosphate (brominated tris), and mixtures or derivatives thereof.

Examples of pigments include iron oxide, carbon black, and mixtures or derivatives thereof.

Examples of lubricants include tetrafluoroethylene, resin powder, graphite, fluorinated graphite, talc, boron nitride, fluorine oil, silicone oil, molybdenum disulfide, and mixtures or derivatives thereof.

Examples of adhesion promoters include silane coupling agents, such as methyltrimethoxysilane, vinyltrimethoxysilane, allyltrimethoxysilane, and 1,6-bis(trimethylsilyl)hexane.

Further additives include siloxane fluids, such as trimethylsilyl- or OH-terminated siloxanes. Such trimethylsilyloxy- or OH-terminated polydimethylsiloxanes typically have a viscosity of 1-150 mPa.s. When present, such siloxane fluids may be present in the liquid curable siloxane elastomer composition in an amount within a range of 0.1% by weight to 20% by weight, based on the total weight of the composition.

Typically, the curable siloxane composition of the present application is in liquid form.

Another aspect of the present application provides an adhesive film, which is formed by the curable siloxane composition of the present application.

The present application further provides a method for preparing the adhesive film, the method comprising curing the curable silicon oxide composition of the present application at a temperature of 10-200°C (e.g., 20-200°C, preferably 50-200°C, more preferably 80-180°C, still more preferably 80-150°C).

Typically, the film can be formed by forming a coating from the curable siloxane composition of the present application in liquid form by a method commonly used in the art (e.g., scrape coating, spin coating, or casting), followed by curing at a temperature of 10-200°C (e.g., 20-200°C, preferably 50-200°C, more preferably 80-180°C, still more preferably 80-150°C) for 1-30 minutes (e.g., 1-20 minutes, preferably 1 -15 minutes, more preferably 1-10 minutes).

The present application further provides a skin care article, comprising a substrate and the adhesive film of the present application.

The substrate in the present application may be any suitable substrate for use in a skin care articles, including, but not limited to, nonwoven fabric, thermoplastic films (e.g., PET film, PE film, PP film, and thermoplastic polyurethane films), thermoplastic polyurethane films, or combinations thereof. In an example of the present application, the substrate is selected from thermoplastic polyurethane, polyester, nonwoven fabric, silicone rubber, or combinations thereof. The adhesive film may be formed directly on the substrate or applied to the substrate by a method commonly used in the art (e.g., bonding, and laminating).

The human skin care article of the present application typically has a water vapor transmission rate (WVTR) of 20-500 g/m² • day, preferably 30-500 g/m² • day, more preferably 30-400 g/m² • day, still more preferably 30-200 g/m² • day, and most preferably 50-150 g/m² • day.

The skin care article of the present application has a peeling force of 0.05-1.5 N/cm (on stainless steel), preferably 0.1-1.2 N/cm (on stainless steel), more preferably 0.15-1.0 N/cm (on stainless steel), and most preferably 0.2-0.6 N/cm (on stainless steel).

In an example of the present application, the adhesive film is further combined with a skin care product, such as a lotion, a serum, a cream, a gel, or a combination thereof. The skin care products include, but are not limited to, for example, hydrating toners, anti-wrinkle serums, whitening lotions, or combinations thereof. By being used in combination with lotions and/or serums commonly used for skin care, the skin care article of the present application can provide various functions, such as skin whitening, combating wrinkles, etc.

The present application further provides a skin care kit, the kit comprising a package of the skin care article of the present application and a skin care product (e.g., a lotion, a serum, a cream, a gel or a combination thereof).

Typically, the skin care kit may further comprise instructions for guiding the user in using the kit. In an example of the present application, the instructions comprise the following steps: applying a skin care product to the skin, and attaching the skin care article of the present application onto the skin.

### Test Methods

The water vapor transmission rate is measured by the upright cup method according to the ASTM-E96 standard.

Peeling force is measured by the method according to the ASTM-D3330 standard.

The moisturizing effect is measured according to the test method in QB/T 4256-2011.

The synergy factor is calculated according to the equation: S = (RR_{A+B+}-1)/(RR_{A+B-}-1)+(RR_{A-B+}-1), where RR_{A+B+} is the effect when A and B are used simultaneously, and RR_{A+B-} and RR_{A-B+} are respectively the effect when A or B is used alone.

### Examples

The present application will be further described below in conjunction with specific examples. The examples of the present application are for a better understanding of the application and do not impose any limitation thereto.

### Examples 1-2 and Comparative examples 1-4

**Table 1**

| | Example 1 | Comparative Example 1 |
|---|---|---|
| Liveo^{™} flexible skin adhesive set A | 50% | 35% |
| Liveo^{™} flexible skin adhesive set B | 50% | 65% |
| Proportion of vinyl after mixing | 0.01-0.08 mol/kg | 0.01-0.08 mol/kg |
| Proportion of hydrogen of silicon-hydrogen bonds after mixing | 0.01-0.08 mol/kg | 0.01-0.08 mol/kg |
| Molar ratio of vinyl : hydrogen of silicon-hydrogen bonds | 1:1 | 2:3 |
| Platinum catalyst | 20 ± 5 ppm | 10 ± 5 ppm |
| Viscosity | 3.1-3.2 Pa.s | 3.4-3.5 Pa.s |
| Peeling force after curing | 0.4 N/cm | about 0 N/cm |

The DuPont^{™} Liveo^{™} 9850 A and B portions were mixed according to the B/A ratios, as shown in Table 1, using a centrifugal mixer to form homogeneous mixtures. The properties of the resulting mixtures are shown in Table 1. The resulting mixtures were scrape-coated onto the substrates, as shown in Table 2, to form coatings with a thickness as indicated in Table 2, and the coatings were cured at 120°C for 5 minutes to form films, and the peeling force was tested immediately. The results are listed in Table 1.

The products obtained in Example 2 and Comparative Examples 1-4 were tested. The results are listed in Table 2 below. Herein, the synergistic effect with L'Oréal CeraVe Moisturising Lotion was tested by the method as follows: first applying the CeraVe Moisturising Lotion to human skin, and then attaching the products of Example 2 and Comparative example 3.

**Table 2**

| | Example 2 | Comparative Example 2 | Comparative Example 3 | Comparative Example 4 |
|---|---|---|---|---|
| Selected substrate | Jiaxing Nanxiong TPU 30 µm | Kevi facial mask sheet | Shanghai Meanlove tencel nonwoven fabric (grammage: 40 g) | Kunshan Sypac PET |
| Thickness of cured siloxane | 400 µm | - | - | 700 µm |
| Water vapor transmission rate | 80 g/m² • day | - | > 500 g/m² • day | 10 g/m² • day |

| Gaining effect on skin moisture when used alone | Providing an appropriate gaining effect on skin moisture for up to 12 hours, see FIG. 1 | Providing a gaining effect on skin moisture only for 2 hours, see FIG. 1 | No gaining effect on skin moisture, see FIG. 1 | Providing a strong gaining effect on skin moisture, not suitable for prolonged use, see FIG. 1 |
|---|---|---|---|---|
| Synergistic effect with CeraVe serum | Calculated synergy factor over 1-12 hours: 1.2-1.7, see FIG 2. | - | No obvious synergistic effect, see FIG. 3 | - |

## Claims

1. A curable siloxane composition, comprising: (a) based on the total weight of the curable siloxane composition, an organosiloxane component having an alkenyl content of 0.01-2 mol/kg; (b) based on the total weight of the curable siloxane composition, an organosiloxane component with the content of hydrogen of silicon-hydrogen bonds being 0.01-2 mol/kg; and (c) a curing catalyst, wherein the molar ratio of alkenyl to hydrogen of silicon-hydrogen bonds is 0.8 or greater.

2. The curable siloxane composition of claim 1, wherein the alkenyl is covalently linked to a silicon atom in the organosiloxane component.

3. The curable siloxane composition of claim 1 or 2, wherein the alkenyl is selected from vinyl, allyl, butenyl, pentenyl and hexenyl groups.

4. The curable siloxane composition of claim 1 or 2, wherein the organosiloxane component (a) may be of the formula below:
(R₁R₂R₃)Si-O-[Si(R₄)(R₅)O]ₘ-[Si(CH₃)(CH=CH₂)O]ₙ-Si(CH₃)(CH₃)(CH=CH₂) (I)
in the formula, R₁-R₅ are each independently selected from alkyl having 1-10 carbon atoms, hydroxyl, aryl having 6-20 carbon atoms, alkenyl having 2-10 carbon atoms (e.g., 2-6 carbon atoms, 2-4 carbon atoms), alkoxy having 1-10 carbon atoms, HON=C-, CH₂(O)CHCH₂OCH₂CH₂CH₂- , -CH₂CH₂CH₂NH₂, -CH₂CH₂CH₂NHCH₂CH₂NH₂, and -OC(O)CH₃; and
m and n each independently represent 0-2000.

5. The curable siloxane composition of claim 1 or 2, wherein the organosiloxane component (a) comprises vinyldimethylsilyloxy-terminated dimethylsiloxane - vinylmethylsiloxane copolymer, vinyldimethylsilyloxy-terminated polydimethylsiloxane, vinylphenylmethylsiloxy-terminated dimethylsiloxane - vinylmethylsiloxane copolymer, and mixtures thereof.

6. The curable siloxane composition of claim 1 or 2, wherein the organosiloxane component (b) may be of the formula below:
(R₆R₇R₈)Si-O-[Si(R₉)(R₁₀)O]ₐ-[Si(CH₃)(H)O]_{b}-Si(CH₃)(CH₃)(H) (II)
in the formula, R₆-R₁₀ are each independently selected from alkyl having 1-10 carbon atoms, H, hydroxyl, aryl having 6-20 carbon atoms, alkoxy having 1-10 carbon atoms, HON=C-, CH₂(O)CHCH₂OCH₂CH₂CH₂-, -CH₂CH₂CH₂NH₂, -CH₂CH₂CH₂NHCH₂CH₂NH₂, and -OC(O)CH₃; and
a and b each independently represent 0-2000.

7. The curable siloxane composition of claim 1 or 2, wherein the organosiloxane component (b) comprises hydrodimethylsiloxy-terminated dimethylsiloxane - hydromethylsiloxane copolymer, hydrodimethylsiloxy-terminated polydimethylsiloxane, hydromethylethylsiloxy-terminated dimethylsiloxane - hydromethylsiloxane copolymer, and mixtures thereof.

8. The curable siloxane composition of claim 1 or 2, wherein the molar ratio of alkenyl to hydrogen of silicon-hydrogen bonds is 0.8 : 1 - 10 : 1, preferably 0.8 : 1 - 8 : 1, more preferably 1 : 1 - 5 : 1, and most preferably 1 : 1 - 2 : 1, for example 1 : 1 - 1.5 : 1 or 0.8 : 1 - 1.2 : 1.

9. The curable siloxane composition of claim 1 or 2, wherein the viscosity at 25°C of the organosiloxane composition before curing is in a range of 0.1 Pa.s to 100 Pa.s.

10. The curable siloxane composition of claim 1 or 2, wherein the catalyst comprises a catalyst selected from platinum group metals or transition metals of the periodic table of the elements, such as platinum, ruthenium, rhodium, palladium, osmium, and iridium; and compounds thereof.

11. The curable siloxane composition of claim 1 or 2, wherein the catalyst is selected from platinum-based catalysts, such as chloroplatinic acid, chloroplatinic acid dissolved in alcohols or ketones and matured solutions thereof, platinum-olefin complexes, chloroplatinic acid-alkenylsiloxane complexes, chloroplatinic acid-diketone complexes, platinum black, carrier-loaded platinum, and mixtures thereof.

12. The curable siloxane composition of claim 1 or 2, wherein the curable composition may also further comprise an inhibitor.

13. An adhesive film formed by the curable siloxane composition of claim 1.

14. A method for preparing the adhesive film, comprising curing the curable silicon oxide composition of claim 1 at a temperature of 10-200°C.

15. A skin care article, comprising a substrate and the adhesive film of claim 13.

16. The skin care article of claim 15, wherein the substrate is selected from thermoplastic polyurethane, polyester, nonwoven fabric, silicone rubber, or combinations thereof.

17. The skin care article of claim 15 or 16, wherein the adhesive film is combined with a skin care product, such as a lotion, a serum, a cream, a gel, or a combination thereof.

18. A skin care kit, comprising a package of the skin care article of claim 15 and a skin care product (e.g., a lotion, a serum, a cream, a gel or a combination thereof).
